# EUROPEAN PATENT APPLICATION

(11) **EP 4 821 513 A1**
(43) Date of publication of application: **14.10.2026**
(21) Application number: 25472005.5
(22) Date of filing: 09.04.2025
(51) Int. Cl.: A61L 9/00

(54) **AUTOMOBILE AROMATIZED DEVICE**

(71) Applicant: Enhance Ltd, 1113 Sofia (BG)
(72) Inventor: Stoimenov, Dimitar, 1113 Sofia (BG)
(74) Representative: Benatov, Samuil Gabriel

(57) **Abstract**

The automobile aromatized device, according to the invention, ensures seamless integration with the vehicle's ventilation system, and employs multiple fragrance cartridges with external controlled selection and blending, offering unparalleled customization and uniform aroma dispersion without permanent vehicle modifications. The device can be installed in easy and stable manner to the existing HVAC system of the vehicle.

## Description

### TECHNICAL FIELD

This invention relates to an automobile aromatizer device, applicable in the automobile industry, and designed to enhance the in-cabin air quality of a vehicle by infusing clean air with a pleasant fragrance before it enters the cabin.

### BACKGROUND OF THE INVENTION

Conventional vehicle air freshening solutions, such as vent-clip diffusers or fragranced cabin filters, often fail to provide consistent fragrance distribution or advanced customization. While some aftermarket products attach to cabin air filters to utilize airflow, they have limited integration and functionality. Built-in fragrance systems in luxury vehicles offer cartridge-based fragrance options but are not available as aftermarket upgrades and lack the flexibility of being installed as an additional feature, having options for external control from a user.

### SUMMARY

A task for present invention is to create an aromatizer for vehicles that ensures seamless integration with the vehicle's ventilation system, and employs multiple fragrance cartridges with external controlled selection and blending, offering unparalleled customization and uniform aroma dispersion without permanent vehicle modifications.

This task is solved by the creation of an automobile aromatized device, comprising a housing, at least one replaceable fragrance cartridge, located in a cartridge seat into the housing, air inlet and outlet ports, wherein the air inlet port is configured for introducing air into the housing to the at least one fragrance cartridge and the air outlet port is configured for releasing fragranced air. Into the housing is arranged a fragrance dispensing system, comprising:
- the air inlet port comprises an internal inlet pipe, connected to an inlet through hole of the housing, wherein the internal inlet pipe has one or more pipe connectors along the length of the pipe, which are connected to inlet valves, connected to inlet openings of each fragrance cartridge,
- the air outlet port comprises an internal outlet pipe connected to an outlet through hole of the housing, wherein the internal outlet pipe has one or more pipe connectors along the length of the pipe, which are connected to outlet valves, connected to outlet openings of each fragrance cartridge,
- a microcontroller for processing commands, connected to a communication module,
- said communication module is configured to receive control commands from external control device for releasing a target fragrance.

Each valve has an electrical motorized actuator that is connected to the microcontroller with ability to receive commands for at least partially opening or closing valves of respective fragrance cartridge.

The electrical motorized actuators, the microcontroller and the communication module are connected to electrical power connectors, configured for connection to a common power source.

In a preferred embodiment of the invention, the space in the fragrance cartridges is gravitationally stratified, with a liquid fragrance in the bottom of the cartridges, and an air gap in the top of the cartridges, configured for passing of air flow from the inlet openings of the cartridges to the outlet openings of the cartridges.

In an alternative embodiment of the invention, each of the fragrance cartridges comprises a porous tampon soaked in fragrance liquid, configured to pass through its pores air flow from the inlet openings of the respective cartridge to the outlet openings of the respective cartridge.

In a preferred embodiment of the invention, the microcontroller is further configured for receiving commands through the communication module for a fragrance volume to be released, and for sending commands to the electrical motorized actuators for an adjustable level of opening of the valves of each fragrance cartridge.

In a preferred embodiment of the invention, an additional adjustable valve is placed on a contact point between the internal inlet pipe and the internal outlet pipe. Said additional adjustable valve is connected to an electrical motorized actuator, connected to the microcontroller with ability to receive commands for complete or partial closing or opening of the additional adjustable valve. The microcontroller is connected to the communication module with ability to receive commands for complete or partial closing or opening of the additional adjustable valve from an external control device. The electrical motorized actuator of the additional adjustable valve is connected to electrical power connector, configured for connection to a power source.

In a preferred embodiment of the invention, the communication module has a processor configured for wireless connection to an external mobile control device for releasing a target fragrance.

In a preferred embodiment of the invention, at least one of the internal inlet pipe and/or the internal outlet pipe have integrated electrical ventilation means, configured to direct air from the inlet through hole to the outlet through hole. Said electrical ventilation means are connected to the microcontroller with ability to receive commands for starting or stopping of ventilation. The microcontroller is connected to the communication module with ability to receive commands for starting or stopping of ventilation from an external control device. The electrical ventilation means is connected to electrical power connector, configured for connection to a power source.

In a preferred embodiment of the invention, the outlet through hole is connected by connection pipe means to an external air outlet pipe, and the inlet through hole is connected to an external air inlet pipe. Alternatively, the internal inlet pipe extends outside the housing through the inlet through hole, and the internal outlet pipe extends outside the housing through the outlet through hole.

The automobile aromatized device can be installed into a vehicle in different embodiments:
- the housing of the automobile aromatized device is fastened inside a HVAC unit of the vehicle between the cabin air filter and the outlet of the HVAC unit directing air into the cabin of the vehicle, and the power connectors of the housing connect the microcontroller, the communication module and the electrical motorized actuators to power system of the vehicle or to an internal battery supply of the automobile aromatized device;
- the housing of the automobile aromatized device is arranged and fastened outside the HVAC unit of the vehicle, and the external air outlet and inlet pipes are connected to a cover of the HVAC unit of the vehicle, having corresponding through holes for tight attachments of said external air outlet and inlet pipes, said through holes are located between the cabin air filter and the outlet of the HVAC unit directing air into the cabin of the vehicle. Preferably, the cover of the HVAC unit of the vehicle can be equipped with deflectors, arranged on the through holes for tight attachments of the extending outside of the housing air outlet and inlet pipes, said deflectors protruding inside the HVAC unit, wherein each deflector consists of connecting part configured for connection to the extending outside of the housing air outlet and inlet pipes, and an end part having a C-shaped cross section, wherein the end part of the deflector connected to the air outlet pipe is open towards the outlet of the HVAC unit directing air into the cabin of the vehicle, and the end part of the deflector connected to the air inlet pipe is open towards the cabin air filter and configured for collecting of passing air.

The automobile aromatized device, according to the invention, introduces customizable fragrances into the car's interior by leveraging the existing HVAC airflow for even distribution. Controlled exclusively via an external control device, it features multiple fragrance cartridges, enabling drivers to select individual aromas or blend them into unique combinations, and adjustable intensity of fragrance, enhancing the driving experience with a tailored olfactory environment.

The device has a compact design, and can be installed in the HVAC system stable and non-invasively.

### Brief description of the figures

Further in the description, the automobile aromatizer device of the invention is explained by a preferred embodiments, given as non-limiting the scope of the invention examples, with reference to the attached figures, where:
Figure 1 shows a preferred embodiment of the invention, wherein the automobile aromatizer device has a replacing cover 7 for the HVAC unit 9.
Figure 2 is a schematic view of the automobile aromatized device according to a preferred embodiment of the invention.

### Exemplary embodiments of the invention

The automobile aromatizer device is configured to be used with the HVAC system of the vehicle.

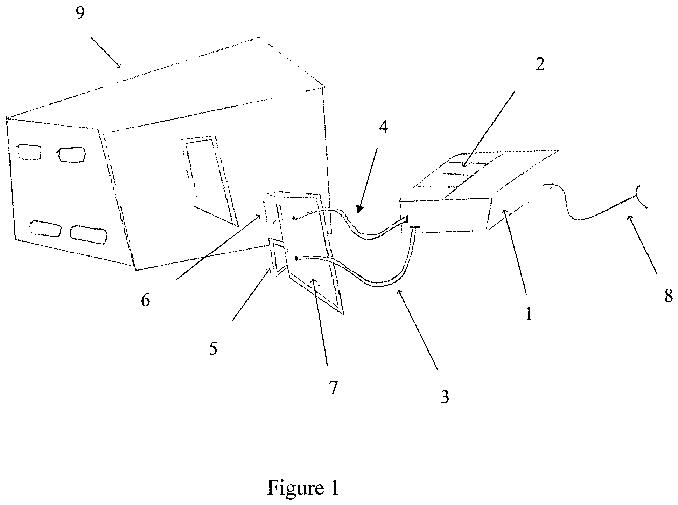

The automobile aromatizer device can be inserted into the cabin filter compartment (HVAC unit), or as shown on figure 1, a housing 1 of the automobile aromatized device can be arranged and fastened outside the HVAC unit 9 of the vehicle, and extending outside of the housing 1 air outlet and inlet pipes 4, 3 of the device to be connected to a cover 7 of the HVAC unit 9 of the vehicle, having corresponding through holes for tight attachments of said extending outside of the housing air outlet and inlet pipes, said through holes are located between the cabin air filter and the outlet of the HVAC unit 9 directing air into the cabin of the vehicle. Thus, the in-cabin air filter cover of the vehicle is replaced, with another cover 7 with same dimensions, fitting in the manufactured fastening means for the cover, and so the device integrates with the vehicle's ventilation system with a non-destructive installation, requiring no permanent alteration to the ventilation system. The device directs a portion of filtered air through the device, and reintroduces fragranced air into the cabin airflow for uniform distribution.

The automobile aromatized device comprises replaceable fragrance cartridges 2, located in a cartridge seat into the housing 1 and air inlet and outlet ports.

The housing 1 is preferably made from durable, heat-resistant ABS plastic or lightweight aluminum to withstand temperature fluctuations. It is compact and aerodynamic to fit within or adjacent to the cabin air filter housing without obstructing airflow.

The air inlet port can include a fine mesh screen to prevent debris from entering the fragrance cartridges.

When the vehicle's ventilation system is active, the aromatizer device draws in clean air through its inlet port. The air passes through the fragrance cartridges 2 and forms a desired scent. The scented air is then released through the outlet port, blending seamlessly with the vehicle's airflow and ensuring a fresh-smelling interior.

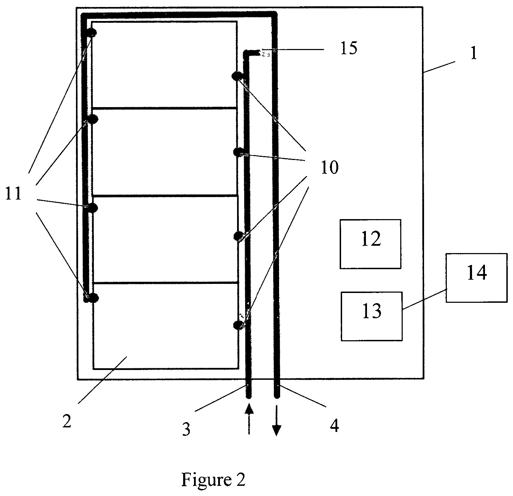

Into the housing 1 is arranged a fragrance dispensing system, as shown on figure 2, comprising:
- the air inlet port, which comprises an internal inlet pipe 3, connected to an inlet through hole of the housing 1, wherein the internal inlet pipe 3 has one or more pipe connectors along the length of the pipe, which are connected to inlet valves 10, connected to inlet openings of each fragrance cartridge 2,
- the air outlet port, which comprises an internal outlet pipe 4 connected to an outlet through hole of the housing 1, wherein the internal outlet pipe has one or more pipe connectors along the length of the pipe, which are connected to outlet valves 11, connected to outlet openings of each fragrance cartridge 2,
- a microcontroller 12 for processing commands, connected to a communication module 13,
- said communication module 13 receives control commands from external control device 14 for releasing a target fragrance.

Each valve 10, 11 has an electrical motorized actuator that is connected to the microcontroller 12 with ability to receive commands for at least partially opening or closing valves 10, 11 of respective fragrance cartridge 2, thus allowing to regulate the volume of air directed through the fragrance cartridges 2, thereby controlling the strength of the distributed aroma.

The electrical motorized actuators, the microcontroller 12 and the communication module 13 are connected to electrical power connectors 8, which are connected to a common power source, such as a small internal battery or a connection to the vehicle's electrical system via a standard connector such as a USB port or 12V outlet, facilitating interaction with an external control device 14.

Since the device includes a plurality of fragrance cartridges 2, each holding a different aromatic substance, the driver is able to select a single fragrance or combine multiple fragrances into a unique aroma profile.

The fragrance cartridges 2 are removable and replaceable, allowing the driver to interchange fragrances based on preference or depletion.

The space in the fragrance cartridges 2 can be gravitationally stratified, with a liquid fragrance in the bottom of the cartridges 2, and an air gap in the top of the cartridges 2, through which air flow passes from the inlet openings of the cartridges 2 to the outlet openings of the cartridges 2. Alternatively, each of the fragrance cartridges 2 can comprise a porous tampon soaked in fragrance liquid, through which pores air flow passes from the inlet openings of the respective cartridge 2 to the outlet openings of the respective cartridge 2.

The device can be controlled exclusively via the control system of the vehicle, or via a logical block of an external control device 14, having a mobile application that wirelessly communicates with the device to select which cartridge(s) the air passes through, adjust fragrance intensity, blend fragrances from multiple cartridges 2, and notify the driver when a fragrance cartridge 2 is low on aromatic substance.

The mobile application enables the driver to activate two or more cartridges simultaneously, adjusting the proportion of each fragrance to create a custom blended aroma, distinct from single-fragrance diffusion.

The device can have an additional adjustable valve 15 or baffle which is placed on a contact point between the internal inlet pipe 3 and the internal outlet pipe 4. Said additional adjustable valve 15 is connected to an electrical motorized actuator, connected to the microcontroller 12 with ability to receive commands for complete or partial closing or opening of the additional adjustable valve 15. The microcontroller 12 is connected to the communication module with ability to receive commands for complete or partial closing or opening of the additional adjustable valve 15 from an external control device 14. The electrical motorized actuator of the additional adjustable valve 15 is connected to electrical power connector, configured for connection to a power source. With the additional adjustable valve 15, it is possible to enable a bypass mode, activated through the mobile application, that allows air to flow through the ventilation system without passing through the fragrance cartridges 2, effectively disabling the aromatizer when no fragrance is desired.

It is possible at least one of the internal inlet pipe 3 and/or the internal outlet pipe 4 to have integrated electrical ventilation means, configured to direct air from the inlet through hole to the outlet through hole, wherein said electrical ventilation means are connected to the microcontroller 12 and able to receive commands for starting or stopping of ventilation. The microcontroller 12 is connected to the communication module 13 with ability to receive commands for starting or stopping of ventilation from an external control device 14. The electrical ventilation means is connected to electrical power connector, configured for connection to a power source.

The outlet through hole can be connected by connection pipe means to extending outside of the housing air outlet pipe, and the inlet through hole can be connected to extending outside of the housing air inlet pipe. These extending outside of the housing pipes can be connected to the internal air outlet and inlet pipes 3, 4, the extending outside of the housing pipes being in this embodiment separate external air outlet and inlet pipes. Alternatively, the internal inlet pipe 3 can be an integral detail, having an internal part into the housing, and an external part extending outside the housing through the inlet through hole, and the internal outlet pipe 4 can be an integral detail, having an internal part into the housing, and an external part extending outside the housing through the outlet through hole, and in this embodiment the external parts of the internal inlet and outlet pipes 3, 4 are the extending outside of the housing pipes.

All over the described herein invention, the feature "pipe" can be replaced with a hose or a suitable tube.

In the embodiment of the invention, wherein the in-cabin air filter cover of the vehicle is replaced, with another cover 7, as shown on figure 1, part of the aromatizer device, the new cover 7 of the HVAC unit 9 of the vehicle can be equipped with deflectors 5, 6, arranged on the through holes for tight attachments of the extending outside of the housing air outlet and inlet pipes, said deflectors 5, 6 protruding inside the HVAC unit 9. Each deflector 5, 6 consists of connecting part configured for connection to the extending outside of the housing air outlet and inlet pipes, and an end part having a C-shaped cross section, wherein the end part of the deflector 6 connected to the air outlet pipe is open towards the outlet of the HVAC unit 9 directing air into the cabin of the vehicle, and the end part of the deflector 5 connected to the air inlet pipe is open towards the cabin air filter and configured for collecting of passing air. The deflectors 5, 6 serve as air diverter mechanisms that channel a small portion of air passing through the in-cabin air filter into the aromatizer, through one or more selected fragrance cartridges 2, and back into the main airflow post-filter zone for even fragrance dispersion throughout the cabin.

It will be clear to those skilled in the art that various modifications of the automobile aromatized device are possible, which are also within the scope of the invention as defined in the appended claims. All parts of the automobile aromatized device can be replaced with technically equivalent elements.

The reference numbers of the technical features are included in the claims solely for the purpose of increasing the comprehensibility of the claims and, therefore, these reference numbers do not have any limiting effect on the interpretation of the elements indicated by these reference numbers.

## Claims

1. Automobile aromatized device, comprising a housing (1), at least one replaceable fragrance cartridge (2), located in a cartridge seat into the housing (1), air inlet and outlet ports, wherein the air inlet port is configured for introducing air into the housing to the at least one fragrance cartridge and the air outlet port is configured for releasing fragranced air, **characterized in that** into the housing is arranged a fragrance dispensing system, comprising:
- the air inlet port comprises an internal inlet pipe (3), connected to an inlet through hole of the housing, wherein the internal inlet pipe (3) has one or more pipe connectors along the length of the pipe, which are connected to inlet valves (10), connected to inlet openings of each fragrance cartridge (2),
- the air outlet port comprises an internal outlet pipe (4) connected to an outlet through hole of the housing, wherein the internal outlet pipe (4) has one or more pipe connectors along the length of the pipe, which are connected to outlet valves (11), connected to outlet openings of each fragrance cartridge (2),
- a microcontroller (12) for processing commands, connected to a communication module (13),
- said communication module (13) is configured to receive control commands from external control device (14) for releasing a target fragrance,
wherein each valve (10, 11) has an electrical motorized actuator that is connected to the microcontroller (12) with ability to receive commands for at least partially opening or closing valves (10, 11) of respective fragrance cartridge (2),
wherein the electrical motorized actuators, the microcontroller (12) and the communication module (13) are connected to electrical power connectors (8), configured for connection to a common power source.

2. Automobile aromatized device according to claim 1, **characterized in that** the space in the fragrance cartridges (2) is gravitationally stratified, with a liquid fragrance in the bottom of the cartridges (2), and an air gap in the top of the cartridges (2), configured for passing of air flow from the inlet openings of the cartridges (2) to the outlet openings of the cartridges (2).

3. Automobile aromatized device according to claim 1, **characterized in that** each of the fragrance cartridges (2) comprises a porous tampon soaked in fragrance liquid, configured to pass through its pores air flow from the inlet openings of the respective cartridge (2) to the outlet openings of the respective cartridge (2).

4. Automobile aromatized device according to any of the preceding claims, **characterized in that** the microcontroller (12) is further configured for receiving commands through the communication module (13) for a fragrance volume to be released, and for sending commands to the electrical motorized actuators for an adjustable level of opening of the valves (10, 11) of each fragrance cartridge (2).

5. Automobile aromatized device according to any of the preceding claims, **characterized in that** an additional adjustable valve (15) is placed on a contact point between the internal inlet pipe (3) and the internal outlet pipe (4), said additional adjustable valve (15) is connected to an electrical motorized actuator, connected to the microcontroller (12) with ability to receive commands for complete or partial closing or opening of the additional adjustable valve (15), wherein the microcontroller (12) is connected to the communication module (13) with ability to receive commands for complete or partial closing or opening of the additional adjustable valve (15) from an external control device (14), wherein the electrical motorized actuator of the additional adjustable valve (15) is connected to electrical power connector, configured for connection to a power source.

6. Automobile aromatized device according to any of the preceding claims, **characterized in that** the communication module (13) has a processor configured for wireless connection to an external mobile control device (14) for releasing a target fragrance.

7. Automobile aromatized device according to any of the preceding claims, **characterized in that** at least one of the internal inlet pipe (3) and/or the internal outlet pipe (4) have integrated electrical ventilation means, configured to direct air from the inlet through hole to the outlet through hole, wherein said electrical ventilation means are connected to the microcontroller (12) with ability to receive commands for starting or stopping of ventilation, wherein the microcontroller (12) is connected to the communication module (13) with ability to receive commands for starting or stopping of ventilation from an external control device, wherein the electrical ventilation means is connected to electrical power connector, configured for connection to a power source.

8. Automobile aromatized device according to any of the preceding claims, **characterized in that** the outlet through hole is connected by connection pipe means to an external air outlet pipe, and the inlet through hole is connected to an external air inlet pipe.

9. Automobile aromatized device according to claims 1-7, **characterized in that** the internal inlet pipe (3) extends outside the housing (1) through the inlet through hole, and the internal outlet pipe (4) extends outside the housing (1) through the outlet through hole.

10. A vehicle with an automobile aromatized device connected to HVAC system of the vehicle, **characterized in that** the automobile aromatized device is according to claims 1-7, wherein the housing (1) of the automobile aromatized device is fastened inside a HVAC unit (9) of the vehicle between the cabin air filter and the outlet of the HVAC unit (9) directing air into the cabin of the vehicle, and the power connectors (8) of the housing (1) connect the microcontroller (12), the communication module (13) and the electrical motorized actuators to power system of the vehicle or to an internal battery supply of the automobile aromatized device.

11. A vehicle with an automobile aromatized device connected to its HVAC system, **characterized in that** the automobile aromatized device is according to any of claims 8 and 9, wherein the housing (1) of the automobile aromatized device is arranged and fastened outside the HVAC unit (9) of the vehicle, and the extending outside of the housing air outlet and inlet pipes are connected to a cover of the HVAC unit (9) of the vehicle, having corresponding through holes for tight attachments of said external air outlet and inlet pipes, said through holes are located between the cabin air filter and the outlet of the HVAC unit (9) directing air into the cabin of the vehicle.

12. A vehicle with an automobile aromatized device connected to its HVAC system, according to claim 11, **characterized in that** the cover (7) of the HVAC unit (9) of the vehicle is equipped with deflectors (5, 6), arranged on the through holes for tight attachments of the external air outlet and inlet pipes, said deflectors (5, 6) protruding inside the HVAC unit (9), wherein each deflector (5, 6) consists of connecting part configured for connection to the external air outlet and inlet pipes, and an end part having a C-shaped cross section, wherein the end part of the deflector (6) connected to the extending outside of the housing air outlet pipe is open towards the outlet of the HVAC unit (9) directing air into the cabin of the vehicle, and the end part of the deflector (5) connected to the extending outside of the housing air inlet pipe is open towards the cabin air filter and configured for collecting of passing air.
